# EUROPEAN PATENT SPECIFICATION

(11) **EP 1 767 653 B1**
(45) Date of publication and mention of the grant of the patent: **25.08.2010**
(21) Application number: 05745933.1
(22) Date of filing: 27.05.2005
(51) Int. Cl.: C12Q 1/68, A61K 45/00, A61P 19/04, C12N 15/09, G01N 33/15, G01N 33/50, G01N 33/53, G01N 33/566, C07K 14/47

(54) **PROBE FOR DIAGNOSIS OF MARFAN'S SYNDROME AND METHOD OF SCREENING WITH THE PROBE**
SONDE ZUR DIAGNOSE VON MARFANS-SYNDROM SOWIE VERFAHREN ZUM SCREENING MIT DER SONDE
SONDE POUR LE DIAGNOSTIC DU SYNDROME DE MARFAN ET PROCÉDÉ DE CRIBLAGE AVEC LA SONDE

(30) Priority: 27.05.2004 JP 2004158099
(43) Date of publication of application: 28.03.2007
(73) Proprietor: Nagasaki University, Nagasaki-Shi Nagasaki , 8528521 (JP); INSTITUT NATIONAL DE LA SANTE ET DE LA RECHERCHE MEDICALE (INSERM), 75654 Paris Cédex 13 (FR)
(72) Inventor: NIIKAWA, Norio, Nagasaki 8528523 (JP); MATSUMOTO, Naomichi, Kanazawa-ku, Yokohama-shi, Kanagawa 2360057 (JP); BOILEAU, Catherine, F-75004 Paris (FR); BEROUD, Gwenaielle, F-34730 Prades-Le-Lez (FR); JONDEAU, Guillaume, F-92130 Issy-les-Molineaux (FR)
(74) Representative: Wibbelmann, Jobst
(86) International application number: PCT/JP2005/010213
(87) International publication number: WO 2005/116253

(56) References cited:
- JP-A- 7 501 212
- DATABASE EMBL [Online] 4 October 2001 (2001-10-04), "Homo sapiens chromosome 3 clone RP11-1024P17, complete sequence." XP002470677 accession no. EMBL:AC096921 Database accession no. AC096921
- DATABASE EMBL [Online] 5 September 2002 (2002-09-05), "Homo sapiens transforming growth factor, beta receptor II" XP002470678 accession no. NM_003242 Database accession no. EMBL:NM_003242 -& OGASA H ET AL: "Cloning of a cDNA encoding the human transforming growth factor-beta type II receptor: heterogeneity of the mRNA" GENE: AN INTERNATIONAL JOURNAL ON GENES AND GENOMES, ELSEVIER, AMSTERDAM, NL, vol. 181, no. 1-2, 28 November 1996 (1996-11-28), pages 185-190, XP004071879 ISSN: 0378-1119
- TANAKA S. ET AL: "A dominant negative mutation of transforming growth factor-beta receptor type II gene in microsatellite stable oesophageal carcinoma" BRITISH JOURNAL OF CANCER, vol. 82, no. 9, May 2000 (2000-05), pages 1557-1560, XP002470825
- KATZKE S. ET AL: "TGGE screening of the entire FBN1 coding sequence in 126 individuals with Marfan syndrome and related fibrillinopathies" HUMAN MUTATION, vol. 20, no. 3, September 2002 (2002-09), pages 197-208, XP002470676
- MIZUGUCHI T. ET AL: 'Heterozygous TGFBR2 mutations in Marfan syndrome.' NAT.GENET. vol. 36, no. 8, August 2004, pages 855 - 860, XP002991035
- COLLOD G. ET AL: 'A second locus for Marfan syndrome maps to chromosome 3p24.2-p25.' NAT.GENET. vol. 8, no. 3, November 1994, pages 264 - 268, XP002991036

## Description

### BACKGROUND OF THE INNOVATION

### 1. Field of the Invention

This invention relates to a probe for diagnosis of Marfan syndrome and a method for determination of whether or not one is affected with Marfan syndrome.

### 2. Related Art

Marfan syndrome is a hereditary disease that affects connective tissues in a body, which shows specific symptoms in many organs such as skeleton, lungs, eyes, heart and aorta. The degree of the symptoms are different among the patients, respectively.

As a gene of patients responsible for Marfan syndrome, Fibrillin 1(FBN1) is well known, but there are many patients which are not accountable by abnormality of the FBN1 gene. It is clinically notable that aorta of a Marfan syndrome patient is larger and more fragile than normal aorta, therefore, there is a concern to cause the dilation of aorta (aneurysm), its dissociation or rupture, and deficiency in function of the aortic valve and mitral valve and so on due to the pressure on the vessel.

However, early diagnosis can be conducted and combined with medical therapies and exercise restriction. Moreover, periodic medical checkup can be also performed, and surgical operations can be conducted depending on their symptoms, thereby, their life duration can be extended to the average life span. As a method of treatment of such Marfan syndrome, a method using extracellular/epithelial growth factor polypeptide has been known, for example (PCT National-phase Japanese Publication No. 2000-508894). Patent Reference Literature: PCT National-phase Japanese Publication No. 2000-508894

In addition, the complete sequence of the Homo sapiens chromosome 3 clone RP11-1024P17 has been submitted to the DDBJ/EMBL/GenBank databases and can be retrieved under accession number AC096921. Further, the complete cDNA sequence and the amino acid sequence of Homo sapiens transforming growth factor, beta receptor II, has been submitted to the DDBJ/EMBL/GenBank databases under accession number NM_003242. However, no link of these sequences with Marfan syndrome has hitherto been established.

### SUMMARY OF THE INVENTION

### (Problem to be solved by the invention)

Even if there is a method for treatment of the disease as mentioned above, there is a problem that it is very difficult to diagnose Marfan syndrome which is a disease of connective tissues with diversity, and its symptoms typically appears in skeleton, lungs, eyes, heart and blood vein. However, all symptoms and features of this disease do not necessarily appear. And even if they appear, the degrees of the symptoms are different among patients. For this reason, there are not a few cases where patients can not recognize their Marfan syndrome at early stage, or patients may consider themselves to be not Marfan syndrome. In general, the most important and effective means to cope with Marfan syndrome is that "to have patients recognize on their Marfan syndrome and to have the patients learn on the correct knowledge of the disease". Namely, since the symptoms such as those appear in patient's cardiovascular systems may progress unconsciously, if a patient does not know on his (her) Marfan syndrome, the symptoms may progress without the patient's recognition, which may cause a serious result. Thus, if a patient can comprehend whether he or she is affected from Marfan syndrome at the early stage, the progression of the disease can be prevented. Despite of it, the only method to diagnose Marfan syndrome at the early stage is genetic diagnosis of FBN1, which is one of the responsible genes for this disease, but abnormality of the gene can not be identified in all patients. This suggests that, in a group of patients, it is suspected that their disease may be caused by some reasons other than abnormality of FBN1.

It is, therefore, an object of the invention to provide a probe useful for diagnosis of Marfan syndrome and screening method using the probe, which can diagnose whether or not a patient is affected from Marfan syndrome caused by other than abnormality of FBN1.

### (Means to solve the problem)

In order to achieve the above object, the inventors made intensive studies on the relationship between Marfan syndrome and its causative genes, which resulted in the finding that the probe described herein can be used for diagnosis of Marfan syndrome. More specifically, the present invention provides a use of a probe for diagnosis of Marfan syndrome as set forth in claim 1 and a method for determined of whether or not one is affected with Marfan syndrome using said probe as set forth in one of claims 8 to 5.

A probe for diagnosis of Marfan syndrome described herein is characterized by using a nucleic acid comprising following (a) or (b), that is, (a) a nucleic acid comprising a base sequence represented by base numbers 1-180000 shown in SEQ ID No.1 of the sequence listing, or (b) a nucleic acid in which a part of the base sequence of said base numbers 1-180000 is deleted, substituted or added, and having 80% homology with said base sequence

Furthermore, a probe for diagnosis of Marfan syndrome described herein, is characterized by using a nucleic acid comprising following (a) or (b), that is, (a) a nucleic acid comprising a base sequence represented by base numbers 1-2090 shown in SEQ ID No.2 of the sequence listing, or (b) a nucleic acid in which a part of the base sequence of said base numbers 1-2090 is deleted, substituted or added, and having 80% homology with said base sequence.

The method according to this invention is characterized by using the probe as described above.

In a preferable embodiment of the method according to this invention, the method is characterized by conducted by the method using nucleic acid hybridization method or using determination of total base sequence.

In a preferable embodiment of the method according to this invention, said method using nucleic acid hybridization method is characterized by being *in situ* hybridization method or Southern hybridization method.

In a preferable embodiment of the method according to this invention, said *in situ* hybridization method is characterized by being fluorescence *in situ* hybridization method.

The method for determination whether or not one is affected with Marfan syndrome is characterized by determining the presence/absence of abscission at the region of p24.1 to p14.2 on the normal chromosome 3, or determining the presence/absence of deletion or point mutation in the nucleic acid comprising a base sequence represented by base numbers 1-2090 shown in SEQ ID No.2 of the sequence listing.

### (The effect of the invention)

The invention is advantageous in that whether or not a patient has Marfan syndrome independent from abnormality of FBN1 can be diagnosed at the early stage of the disease. For example, in a patient who has a mutated gene but does not show any symptoms, diagnosis can be made before onset of the disease and medical treatment can be conducted at the early stage, which may result in improved prognosis of the disease.

Furthermore, since abnormality of FBN1 constituting extracellular matrix and abnormality of TGFB signal transduction system are confirmed in similar human disease, this invention may lead to development of a new therapeutic method for this disease which is targeted to TGFB signal transduction system.

### BRIEF DESCRIPTION OF THE DRAWINGS

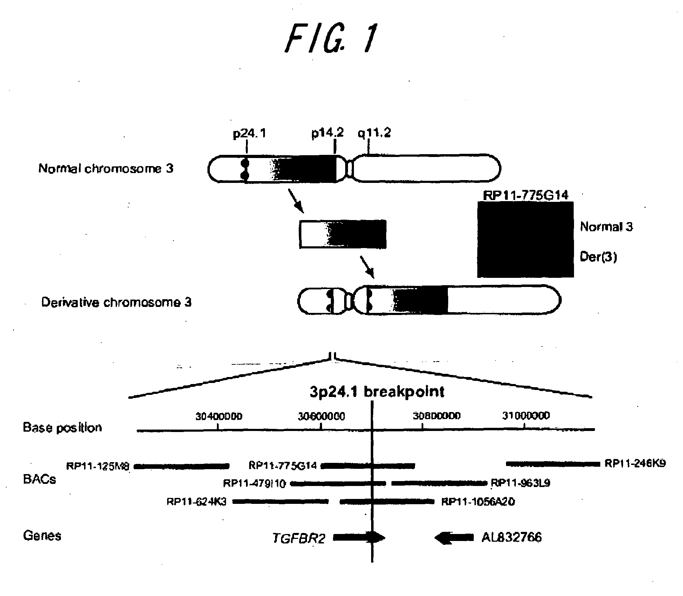

Figure 1 shows the vicinity of TGFBR2 isolated from 3p24.1 of a Japanese patient having a complex chromosomal abnormalities. The 3p24.1 breakpoint was analyzed because the Marfan syndrome type 2 responsible locus (MFS2 locus) (MLCTD locus) was also mapped to 3p24.2-p25. In this individual, the chromosomal segment 3p24.1-p14.2 was inserted into 3q11.2. FISH analysis confirmed that RP11-775G14 spans the 3p24.1 breakpoint. A summarized physical map covering the 3p24.1 breakpoint is indicated. Horizontal bars show BAC clone; clone spanning the breakpoint; and the arrow shows the gene. TGFBR2 is only the gene mapped to the breakpoint.

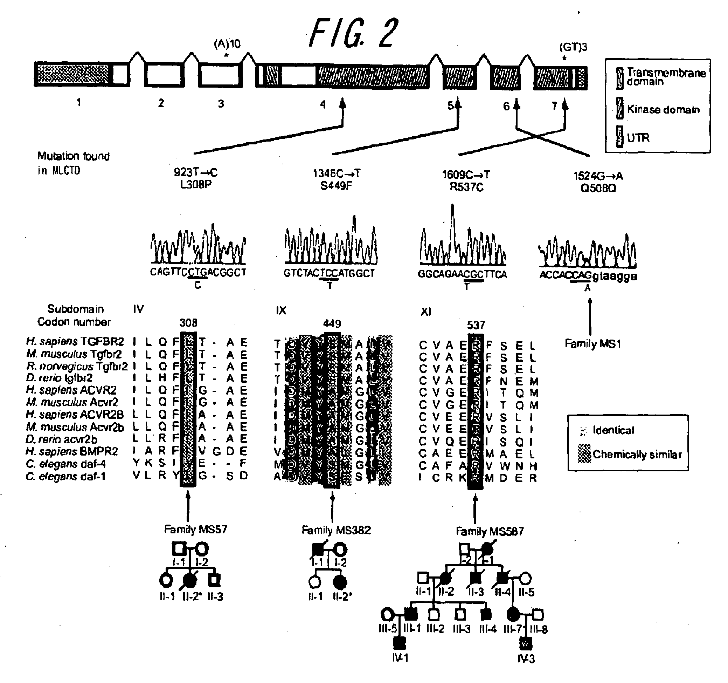

Figure 2 shows haplotype analysis of a large French family (MS1) and mutant causing abnormal splicing.
a: Pedigree of family MS1 and segregation of 3p24.1 markers in members affected with MFS2 (MLCTD (black symbols) or unaffected (white symbol). Green symbols indicate mutants, members of c.1524g>A, orange symbols indicate members without the mutation. The halotype cosegregating with the disease is shown in red.
b: Normal (green) and abnormal splicing (orange) were caused by c1524 G>A (p.q508Q). In the mutated allele, 23 bp nucleotides of intron 6 were newly added after exon6 and connected to regular exon 7, resulting in a premature stop codon at amino acid 525.

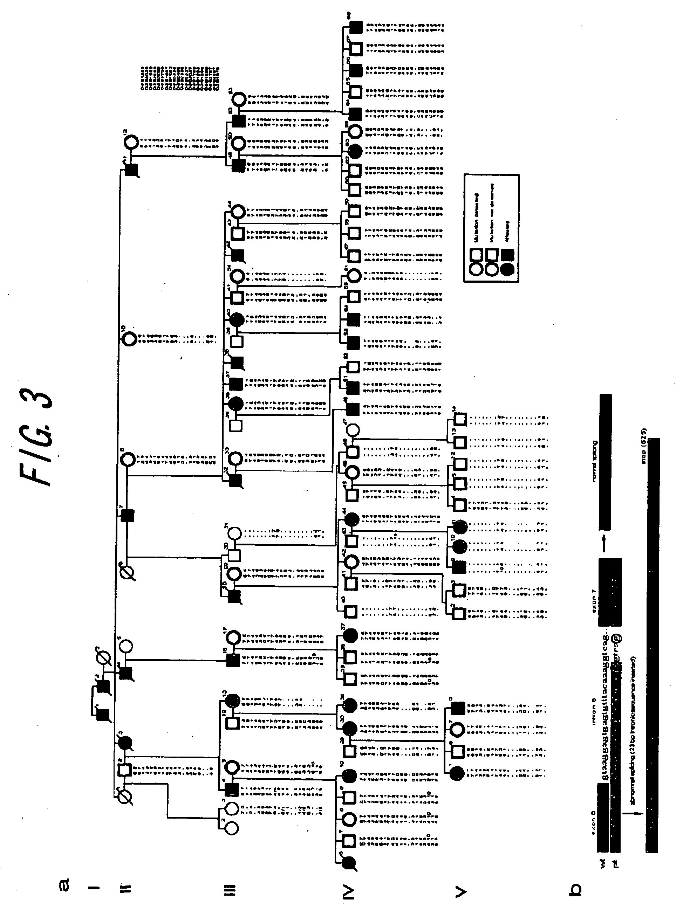

Figure 3 shows genomic structure of TGFBR2 and mutations found in MFS2 (MLCTD). TGFBR2 consists of seven exons. The squares show the exons. A transmembrane domain, a kinase and the untranslated regions are shown in light blue, pink and gray, respectively. (A)10 and (GT)3 are genomic instability sites. Three other missense mutations, c.923T>c (p.1308p), c.1346c>T (p.S449), and c1690c>T (p.r537c) are found in families MS57, MS382 and MS587. Each mutation occurred at an amino acid that is evolutionally conserved or chemically similar in a kinase domain among mouse and rat Tgfbr2, mouse and zebrafish Acvr2, and nematode daf genes. Multiple sequence alignment was done using web base software CLUSTALW.

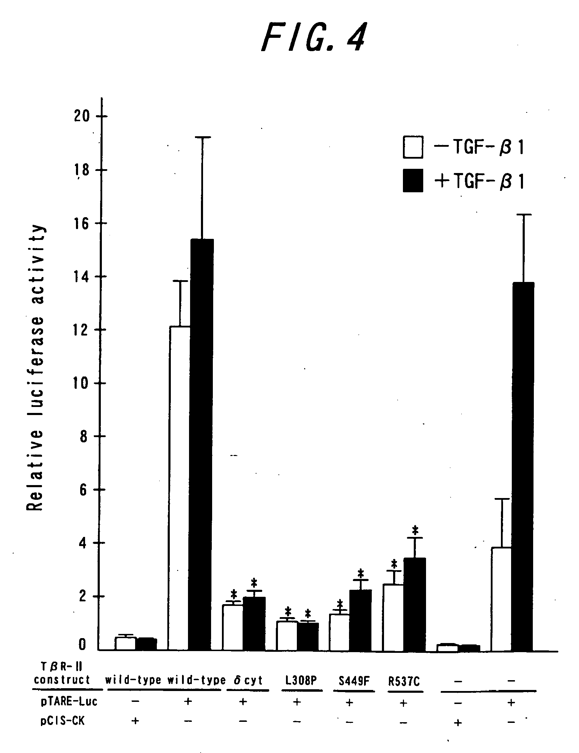

Figure 4 shows impairment of TGF-B signaling activity by TGFBR2 missense mutations in individuals with MFS2 (MLCTD).
Relative luciferase activity (RLA) of pTARE-Lux cis-reporter in HER293 cells after transient transfection with various TGFBR2 constructs was calculated by normalization using the activity of cotransfected control vector, pRL-TK, containing distinguishable R.reinformis Luciferase. Data represent mean + s.d. Cells were incubated 44 hours with or without exogenous TGF-β1 (10ng.ml) (R&D, Minneapolis, MN). Basal activity only by pTARE-Lux cis-reporter probably due to exogenous TGF-β1 in HEK 293 cells. Transfection with wild-type TGFBR2 resulted in a high PLA (about 16), whereas transfection with the truncated mutant lacking the kinase domain, d cyt, gave a significantly lower RLA. Transfection with other missense mutants, L308P, S449F and R537C, also gave significantly lower RLAs, similar to that seen with transfection of d cyt.

### BEST MODE FOR CARRYING OUT THE INVENTION

A probe for diagnosis of Marfan syndrome described herein uses a nucleic acid comprising following (a) or (b), that is, (a) a nucleic acid comprising a base sequence represented by the base numbers 1-180000 shown in SEQ ID No.1 of the sequence listing, or (b) a nucleic acid in which a part of the base sequence of said base numbers 1-180000 is deleted, substituted or added, and having 80%, preferably 90%, more preferably 95% homology with said base sequence. Said nucleic acid mentioned above is derived from TGFBR2 of human chromosome 3 and the nucleic acid is complementary to said TGFBR2. Concretely, the nucleic acid is complementary to a genomic DNA including the exon 1-7 and the intron of TGFBR2. The nucleic acid that can be used as the probe according to the invention includes a nucleic acid in which a part of said base numbers 1-180000 is deleted, substituted or added, and having 80%, preferably 90%, more preferably 95% homology with said base sequence. It means that even if one in which a part pf the sequence is deleted, substituted or added could be used, for example, as a probe, as described below. For example, as described below, when one intends to diagnose whether a patient is affected with Marfan syndrome or not, the presence/absence of abscission at the region of p24.1-p14.2 on the chromosome 3 is important. Therefore, a sequence having such region and exhibiting a homology higher than a determined level could be used as a probe.

Moreover, a probe for diagnosis of Marfan syndrome described herein is a nucleic acid comprising following (a) or (b), that is, (a) a nucleic acid comprising a base sequence represented by the base numbers 1-2090 shown in SEQ ID No.2 of the sequence listing, or (b) a nucleic acid in which a part of the base sequence of said base numbers 1-2090 is deleted, substituted or added, and having 80%, preferably 90%, more preferably 95% homology with said base sequence. Therefore, even a sequence having such homology could be also used as a probe, as described below.

The method for purification, isolation of the aforementioned nucleic acids will be described below. The methods for said purification and isolation are not particularly limited, the nucleic acid can be purified and isolated according to the following procedure.

Genomic DNA can be extracted from peripheral lymphocytes using a standard protocol. The exon (GenBank accession number, NT022517) covering the coding region of TGFBR 2 can be amplified directly by PCR method for the purpose of sequencing. The PCR products can be obtained by repeating PCR reaction, for example, by amplifying 25-45 times at about 95°C for 10-300 seconds, at 50ºC for 10-300 seconds, and at about 72ºC for 10-300 seconds, in a 50 µl mixture containing a proper buffer solution, such as 1 x PCR buffer (Applied Biosystems, Foster city, CA) containing 1.5 mM MgCl₂, 0.2 ml of each dNTP, 1 µMU, TaqDNA polymerase. After purification of the obtained PCR product, its base sequence can be determined according to a standard method, for example, by Sanger method.

It also is possible to extract total mRNA from human fibroblast cells. The mRNA obtained can be reverse-transcribed by reverse-transcriptase to obtain DNA.

By using the probe thus obtained, the method according to this invention can be performed as below. In a preferred embodiment, the method can be performed by a method using nucleic acid hybridization method or by a method using total base sequence determination. As to a method utilizing said nucleic acid hybridization, *in situ* hybridization method or a Southern blotting method can be mentioned.

As a method to use the probe described herein, the above-mentioned nucleic acid can be amplified directly or by PCR method, which can be used as a probe. A genomic DNA from a patient may be digested by a proper restriction enzyme, then the digested DNA may be immobilized by blotting on a polymer membrane, and then the probe described herein can be subjected to hybridization. The method for hybridization is not particularly limited by conventional methods, however, Southern blotting method, *in situ* hybridization method and base sequence determination method can be listed, for example. The *in situ* hybridization method is preferable, because the method enables rapid and accurate screening. The *in situ* hybridization method may include fluorescence *in situ* hybridization method (hereinafter referred to as FISH method), radioisotope *in situ* hybridization method and the like. In summary, the FISH method generally comprises, for example, preparing a chromosome sample on a slide glass, hybridizing it with a labeled probe, and conducting direct investigation by a microscope.

As a support medium used for hybridization of the probe described herein , not only a thin film, a powder, a particulate matter, a gel, a bead, and a fiber, a dispersion liquid and emulsion and the like may be also listed. They may be filled into an adequate column for utilization. Among them, a thin film such as a nitrocellulose film and a nylon film are preferable.

An example of the labeling used for the probe described herein will be explained. For the examples of the labeling, those known among the skilled artisan can be used, and not particularly limited. For example, a radioactive atom such as ³²P and ³⁵S, biotin group, avidin group, or enzyme labeling, fluorescence labeling and the like can be used. In addition, in the case an antigen-antibody system is used, it may include antigens. Such embodiment is also included within the scope of the invention.

As to the probe, the nucleic acid binds complementarily with a part of normal chromosome 3. However, if the chromosome 3 has some abnormality such as deletion, the nucleic acid described herein dose not bind with the abnormal chromosome 3. Utilizing such nature that it fails to binding with the chromosome 3, the probe can be utilized in this invention.

When one is to diagnose whether one is affected with Marfan syndrome or not, it is primarily important to compare each base sequences determined by base sequencing. Otherwise, it is also important to observe a large deletion or abscission using Southern method or FISH method.

Utilizing the property described above, it is possible to diagnose whether or not one is affected with Marfan syndrome. That is, by using the method described above, it is possible to determine the presence/absence of deletion or abscission at the region of p24.1 to p14.2 on the normal chromosome 3, or the presence/absence of deletion or abscission in the nucleic acid comprising a base sequence represented by base numbers 1-2090 shown in SEQ ID No.2 of the sequence listing.

### EXAMPLE

An example of the invention will be explained, however, this invention is not intended to limited within the example. The example explained below is used to present an embodiment of this invention, but this in not intended to exclude any modifications or alterations as long as it does not deviate from the abstract and the range of this invention described in the claims.

### Example 1

Marfan syndrome is a disease of extra-cellular matrix that has cardinal expression in eyes, skeleton and cardiovascular, and involved in the deficiency of 15q21.1 on fibrin gene (FBN1). The inventors have previously mapped a gene locus of 3p24.2-p25, also known as MFS type 2 (MFS2OMIM154705), in a large French family (MS1 family). The chromosome breakpoint of 3p24.1, that divides TGFB receptor 2 gene (TGFBR2) was identified in Japanese Marfan syndrome patient, from which the inventors considered the TGFBR2 to be MFS2 gene. The inventors found p.Q508Q mutant of TGFBR2, which occurred by abnormal splicing and having abscission on MS1 in Japanese patients. The three other missense mutants were found in other four patients not having blood relationship with the family, and deficiency in the function of TGFB signal activity was determined by luciferase measurement.

The inventors encountered a Japanese patient with complex chromosome rearrangements, 46,XY,t (1;5;4) (p35;q33.2;q35), ins(3)(q11.2;p24,1p14.2) de novo. As one of the chromosome breakpoints, 3p24.1 was consistent with the MFS2 locus, therefore, it was hypothesized that disruption of TGFBR2 gene at 3p24.1 by chromosome by insertion caused the disease. FISH mapping revealed that BAC clones, RP11-479i10, RP11-775G14 and RP11-1056A20 spanned the breakpoint (Fig.1). The TGFBR2 gene was the only gene in the common region overlapped by the three BACs, and was highly likely to be disrupted at the breakpoint (Fig.1).

The TGFBR2 was analyzed in a large French family (MS1) that provided MFS2 mapping at 3p24.2-p25. Among a total of 74 members from MS1 analyzed, c.1524>A substitution was found in 25 members affected with MFS2, but absent in 32 healthy sibs, 16 unrelated spouses, one suspected to be "affected" (IV-53) and 60 unrelated French healthy controls (Fig 2a). This transition did not induce amino acid substitution (p.Q508Q), but located at the last nucleotide of exon 6, implying that a splicing process could be affected. The consensus value on splicing was modified from 95.6 to 82.3 by c.1524G>A and another good consensus sequence (Aggt xxg, value 90.5) was located 23 bp away in the intronic sequence (Fig.2b). RT-PCR analysis with a primer set in exons 5 and 7 (MUT1 F and MUT1 B) using cDNA of fibroblasts from two affected members revealed a larger product together with the expected normal band (data not shown). Sequencing analysis confirmed an abnormal splicing by which 23 bp nucleotides of intron 6 were added to exon 5 creating a premature stop codon at amino acid position 525 (Fig.2b). This abnormal fragment was not found in fibroblast from III-41 (healthy) or unrelated normal control. Molecular data were consistent with clinical data.

The inventors studied 9 MFS probands from 9 unrelated French families, as well as 10 unrelated Japanese MFS patients, in which no FBN1 mutation or no linkage to FBN1 was recognized. After bi-directional sequencing of all the 7 exons of TGFBR2, the inventors identified three missense mutations: c.923T>T (p.L308P), c.1346C>T (p.S449F) and c.1609C>T (p.R537C). The c.923T>C was found only in the proband of French family MS57 but not in her unaffected parents (paternity confirmed) or in two sibs, thus demonstrating de novo occurrence. The c.1346C>T was identified in a French family (MS382), and c.1609C>T in another family (MS587) and in one Japanese patients. In MS587, two affected members (IV-1 and III-4) were also shown to carry c.1609C>T. All the missense mutations are located within the serine/threonine kinase domain of TGF-β receptor 2 protein, and each affects an amino acid that is highly conserved or chemically similar in the homologous mouse and rat Tgfbr2 gene, mouse and fly Acvr2, and nematode daf genes (Fig.3). Among 267 unrelated Japanese healthy controls (534 chromosomes) and 92 unrelated Caucasian healthy controls (184 chromosomes), no mutation was found. The inventors also investigated 10 French probands with thoracic aortic aneurysms and dissession (TAAD), because a locus with putative associated with this disease has been mapped at 3p24-25, but identified no mutations.

The inventors also used an in vitro luciferase assay to evaluate the effect of the mutations on TGF-β signaling. The inventors transfected a pTARE-Luc cis-reporter plasmid and a pRL-TK vector into HEK 293 cells and determined relative luciferase assay (RLA). The inventors detected basal activity that increased threefold after exposure to exogenous TGF-β1 (Fig.4a). Transfection with wild-type (WT) TGFBR2 cDNA resulted in RLA of 12 even in the absence of exogenous TGF-β1 as previously reported. In contrast, RLA decreased significantly in the truncated mutant d cyt, lacking the kinase domain, even with exogenous TGF-β1. Transfection with the other missense mutants, L308P, S449F, and R537C, also caused significant decrease in RLA, indicative of their negative effect on TGF-β1 signaling.

TGF-Bs are cytokines that regulate many cellular process, including proliferation, cell cycle arrest, apoptosis, differentiation and extracellular matrix formation, through a heteromeric complex of type I and type II receptors with serine-threonine kinase activities in their cytoplasmic domains. Defective TGF-B signaling transduction is important in tumorigenesis, and TGFBR2, SMAD4 and SMAD 2 act as tumor supressors in several cancers. Most mutations in TGFBR2 found in tumor cells are clustered in the poly-A repeat in exon 3 (Fig.3). Many missense mutations are associated with various cancers and have been suggested to result in loss of function. Only one germline mutation (c944C>T located in the kinase subdomain of the receptor (p.T315M)) has been reported in a kindred with hereditary nonpolyposis colorectal cancer. Functional studies indicated that defection in growth inhibition in response to TGF-B and c.944C>T maintained the ability to induce extra-cellular matrix proteins, suggesting that two divergent TGF-B signaling transduction pathway may exist. This may partially explain why malignancies were not observed in MFS2 (MLCTD) with the TGFBR2 mutations. Moreover, as the inventors identified three classes of TGFBR2 mutation, loss of function of TGFBR2 probably accounts for the dominant inheritance of the disease. Neptune et al. verified excessive TGF-β activity in Fbn-1 deficient mice that probably underlies their tendency to develop emphysema and could explain other manifestations of Marfan syndrome. The inventors previously reported that domain specific germ-line mutations of TGFB1 cause Camurati-Engelmann syndrome (OMIM #131300) and that affected individuals also usually have Marfanoid habitus (long slender extremities and vertebral deformation). TGF-β probably regulates the extra-cellular matrix. The inventor's results provide further evidence that perturbation of TGF-β signaling contributes to the pathogenesis of extracellular matrix disorders.

Among the ten French Marfan syndrome probands examined, only four had mutations in TGFBR2, and these four individuals share a common clinical description: prominent aortic, skeletal and skin/integument anomalies; mild oculat anomalies (except individual MS1-IV-83, who has ectopia lentis); infrequent dural ectasia; and pulmonary abnormalities. Further data must be collected to assess the complete clinical spectrum associated with mutations in this locus. Other overlapping pathologies, such as MASS syndrome (mitral valve prolapse, aortic dilartation, and skin and skeletal manifestations syndrome, OMIM #604308), isolated familial mitral valve prolapse (OMIM #157700) and autosomal dominant TAAD (OMIM #132900), should also be investigated. Although no mutation was found in the ten TAAD probands that the inventors tested, the disease is heterogeneous and further investigations are warranted before ruling out the possibility that TGFBR2 and TAAD2 are allelic.

### Subject and clinical evaluation

A 13-year-old Japanese boy with 46, X,Y,t(1;4;5) (p35;p33.2q35), ins (3) (q11.2;p24.1p14.2) de novo. He underwent an operation for severe pectus excavatum at 12 year of age. He was clinically diagnosed with Marfan syndrome because of his high arched palate, arachnodactyly (positive wrist and thumb sings), atlantoaxial subluxation, scoliosis, reduced extensions at the elbow, dilatation of the ascending aorta involving the Valsalva sinus, mitral valve prolapse, incomplete right bundle-branch block, bilateral inguinal hernia. He had been treated with growth hormone for pituitary short statue. At the age of 12, his length was 135.2cm (-2.0 s.d.) and weight 24.8kg (-2.0 s.d.).

### Family MS-1

This large French family was identified after the death of a 39-year-odl male family member from aortic dissection. The complete clinical features of individuals were described in the first part of the family study previously. The second part of the family study was done in the Marfan Clinic of Hospital Ambroise Pare. Individuals at risk underwent careful physical examination, echocardiogtaphy and slit-lamp examination. Twelve new members were evaluated and gave samples (individuals IV-40, IV-43, IV-48, V-6, V-7, V-8, V-9, V-10, V-11, V-12, V-13 and V-14), four patients with degraded DNA gave samples again (III-13, III-41, IV-32 and IV-53). As a result, among 12 family members, 8 individuals without abnormality in any of the skeletal, and cardiovascular systems, or with only isolated minor findings in these organs or tissues, were considered unaffected,

### Family MS-57

MS57-MA319 presented with dolichostenomelia, pectus carinatum, arachnodactyly, spondylolisthesis, protrusio acetabulae, dysmorphic face, narrow arched palate and dental crowding, striae distensae, flat cornea, aortic dulation (+8 s.d) with mild regurgitation, mitral valve prolapse and myxoid valves, aneurysm of interatrial septum and small atrial septal defect and was diagnosed with Marfan syndrome. She died suddenly at age 18 of an unknown cause. Her brother and parents were unaffected. Her sister, MS-MA328, presented with only arachnodactyly and mild pectus carinatum but no other feature of Marfan syndrome. She daughter of age 6 is unaffected.

### Family MS-382

MS382-MA1515 is a 10-year-old girl who was diagnosed with Marfan syndrome in infancy. Her farther died suddenly at the age of 39. She had pronounced skeletal symptoms of Marfan syndrome, including asymmetric pectus carinatum, arachnodactyly with positive wrist sign, dolichocephaly, severe dorso-lumber scoliosis, hyperlaxity, joint hypermobility at ankles and knees, clear muscular hypotonia and umbilical hernia. Radiological examination showed major coax valga and absence of dural ectasia. She presented with patent ductus arteriosus, foramen ovale, interventricular septal defect and aortic root dilation (50 mm at age 10) requiring surgery at age 12. No ocular symptom was noted, except for very slow dilation. Her mother and sister were alive and showed no features of MFS2 (MLCTD).

### Family MS-87

MS587-MA1771 (III-7) is a 31-year old female who had been examined after her father's (II-4) sudden death at the age of 26. She had aortic dilation and mitral valve regurgitation at the age of 5, and dilation was +6 s.d. at aged 31. She presented with a history of lung disease and pleurisy, narrow palate with dental crowding, scoliosis, striae distenasae and mild myopia with mild astigmatism. Family history identified two other sudden deaths: her father's brother (II-3) at age 26 and her father's sister (II-2) at aged 32. Individual III-1 (age 34) showed aortic dilation, reduced upper to lower segment ratio, dolichostenomelia, pectus excavatum, narrow arched palate and scoliosis. Individual III-4 (age 28) had mild mental retardation, mild scoliosis, pectus excavatum, hyperlaxity and hypermobility of joints, high narrow palate with dental crowing, pes planus, striae distensae, and aortic dilation (+6 s d.) with moderate aortic valve regurgitation. Individual IV-1 (age 4.5 years) presented with aortic dilation (+6 s.d.) without regurgitation, increased body length, dolichocephaly, narrow arched palate and mild scoliosis.

### Japanese patient

Japanese individuals with Marfan syndrome who were shown not to carry FBN1 mutations were screened for TGFBR2 mutations. Seven of the individuals were previously described. All ten individuals fit the revised criteria for Marfan syndrome, although detailed clinical information was not available according to a protocol approved by the Institutional Review Board of the National cardiovascular Center Research Institute.

### Mutation analysis

The inventors extracted genomic DNA from peripheral blood lymphocytes using standard protocols. The inventors amplified exons (GenBank accession number, NT 022517) covering the TGFBR2 encoding region by PCR for direct sequencing. PCR amplification and primers sequences are available on request. PCR was repeated 35 cycles of 95°C for 30s, 50°C for 30s, and 72°C for 30s in a 50 µl mixture, containing 1 X PCR buffer with 1.5 mM MgCl₂, 0.2 mM each dNTP, 1 µl each primer and 2.5U TaqGold polymerase (Applied Biosystems, foster city, CA). The inventors amplified PCR products with ExoSAP-IT (Amersham-Pharmacia, Cleveland, OH) and sequenced both strands with BigDye Terminator chemistry version 3 by the standard protocol. Sequencing reactions were carried out at 96°C for 10s, 50°C for 5s and 60°C for 4 min (25 cycles) on Gene Amp PCR System 2700 or 9700 (Applied Biosystems). The inventors purified the reaction mixture and analyzed it on the ABI Genetic Analyzer 3100 (Applied Biosystems) according to the supplier's protocols with the sequence analysis softwere (Applied Biosystems) and the AutoAssembler version 2.1.1 software (Applied Biosystems).

The inventors extracted total mRNA from human fibroblast of two affected (Individuals III-37 and IV-10) and one unaffected (Individual III-41) members of family MS1, and a normal control person, using RNA B (Registered Trademark, Bioprobe Systems, Montreuil-sur-bois, France) according to the manufacture's instructions.

The inventors treated samples with RQ1 RNase-Free DNase (Promega, Madison, WI, USA) and reverse-transcribed total mRNA using Superscript II RNase Reverse Transcriptase (Registered Trademark, Gibco BRL/Invitrogen, Cergy Pontoise, France). The inventors carried out PCR analysis with a primer set in exon 5 and 7 and then sequenced the DNA as previously described (MUT1 F: 5'-TGC AAG ATA CAT GGC TCC AG-3'; MUT1 B: 5'-AGC TCA CTG AAG CGT TCT GC-3'). The inventors sequenced samples from all subjects from French families by PCR, using primer for MS1 MUT2F and MUT2B (MUT2 f: 5'-TGT GTC GAA AGC ATG AAG GA-3', MUT2B:5'-TCC AGA ATT CTC TGC CAC CT-3'), and MUT3F and MUT3B for MS57 (MUT3F: 5'-TGC CTC TTG GAA GAC AGC GAA CT-3'; MUT3B : 5'ACT CCT GTA GGT TGC CCT TG-3').

### Genotyping of family MS1

The inventors selected microsatellite markers and tetranucleotide markers from public genetic database (Genethon:http://wwwgenethon.fr/, and CHLC:http://www.chlc.org/.). The genotype of genomic DNA was as previously described. The inventors constructed regional halotypes for 20 marker loci from tel-D3S1293 to D3S1619-en (Fig.2; D#S1293, D2S3038, D3S1599, D3S3659, D3S3598, D3S3700, D3S1567, D3S1583, D3S2336, D3S2466, D3S2335, D3S2337, D3S3037, D3S1759, D3S1283, D3S1266, D3S1609, D3S3727, D2S3567 and D3S1619). D3S3567 is an intragenic TGFBR2 marker. Physical mapping combined with halotype allowed the inventors to determine the marker order.

### DNA constructs

The inventors generated wild-type TGFBR2 cDNA (GenBank accession number, NM003242; amino acids 1-567) and truncated cDNA (d cyt: amino acids 1-222, lacking the entire kinase domain) by RT-PCR using human fetal brain BD Marathon-Ready cDNA (BD Biosciences, Palo Alto, CA) as a template and subcloned them into the pcDNA3.1(-) expression vector (Invitrogen, Carlsbad, CA). The inventors carried out site-directed mutagenesis using the QuickChange Site-directed Mutagenesis kit (Stratagene, LaJolla, CA) according to the manufacture's protocol to generate three variant types of TGFBR2 in pDNA3.1(-), 1308P, S449F and R537C, which were found in the MFS2 (MLCTD) families that the inventors studied. All variant cDNAs were verified by sequencing. Primer sequences are available on request.

### Cell culture, transfection and luciferase assay

The inventors grew HEK 293 cells in Dulbecco's modified Eagle's medium (DMEM: Sigma, St.Louis, MO) containing 10% fetal bovine serum at 37°C in a 5% CO₂ incubator. The inventors transiently transfected HEK 293 cells with a TGFBR2 construct (wild-type, d cyt, L308P, S449F or R537C), a reporter plasmid (pTARE-Luc cis-reporter or pCIS-CK negative control) and a pRL-TK vector (an internal control for standardization) in Dual-Luciferase Reporter Assay System (Promega) using Transfast Transfection Reagent (Promega). The pTARE-Luc cis-reporter plasmid contains the basic promoter element (TATA-box) and TGF-β/Activin response element (TARE). This reporter plasmid expresses firefly luciferase under control of these elements, whereas the pCIS-CK negative control plasmid contains no inducible cis-element to evaluate whether effects are TGF-β signaling specific. After transfection, the inventors incubated HEK 293 cells in DMEM for 36 h and then replaced the medium with DMEM containing 10 ng/ml of TGF-β1. The inventors collected cells 8 h later and assessed them for lucifarase activity in quadruplicate. The inventors measured luciferase activity using TD-20/20 Luminometer DLReady (Turner Designs Instrument, Synnyvale, CA). Statistical analysis was done by post hoc test using StatView and P<0.05 was considered statistically different.

### (Industrial Applicability)

For instance, it is possible to improve prognosis of the disease, by diagnosing a patient having mutated gene at the early stage before onset of the disease, and conducting medical treatment. Moreover, as abnormality of FBN1 constituting extracellular matrix and TGFB signaling systems were confirmed in similar human diseases, it is expected that this invention will lead to development of therapies targeted to the TGFB signaling systems, against diseases of this group. Therefore, it is expected that this invention will contribute in wide fields such as medicine, biochemistry, biology and molecular biology.

## Claims

1. Use of a probe for diagnosis of Marfan syndrome, the probe being
(1) a nucleic acid comprising following (a) or (b):
(a) a nucleic acid comprising a base sequence represented by base numbers 1-180000 shown in SEQ ID No.1 of the sequence listing, or
(b) a nucleic acid in which a part of the base sequence of said base numbers 1-180000 is deleted, substituted or added, and having 80% , homology with said base sequence, or
(2) a nucleic acid comprising following (a) or (b):
(a) a nucleic acid comprising a base sequence represented by base numbers 1-2090 shown in SEQ ID No. 2 of the sequence listing, or
(b) a nucleic acid in which a part of the base sequence of said base numbers 1-2090 is deleted, substituted or added, and having 80% homology with said base sequence.

2. A method for determination of whether or not one is affected with Marfan syndrome using the probe as defined in claim 1, **characterized by** determining the presence/absence of deletion or abscission at the region of p24.1 to p14.2 on the chromosome 3, or by determining the presence/absence of deletion or point mutation in a nucleic acid comprising a base sequence represented by base numbers 1-2090 shown in SEQ ID No. 2 of the sequence listing.

3. The method according to claim 2, which is conducted by a method using nucleic acid hybridization method or by a method using determination of total base sequence.

4. The method according to claim 3, wherein said method using nucleic acid hybridization method is *in situ* hybridization method or Southern hybridization method.

5. The method according to claim 4, wherein said *in situ* hybridization method is fluorescence *in situ* hybridization method.

## Patentansprüche

1. Verwendung einer Sonde zum Diagnostizieren des Marfan-Syndroms, wobei die Sonde
(1) eine Nukleinsäure, umfassend die folgenden (a) oder (b):
(a) eine Nukleinsäure, umfassend eine Basensequenz, die durch die in der SEQ ID Nr. 1 des Sequenzprotokolls gezeigten Basennummern 1-180000 dargestellt ist, oder
(b) eine Nukleinsäure, in der ein Teil der Basensequenz der genannten Basennummern 1-180000 deletiert, substituiert oder addiert ist, und die 80% Homologie mit genannter Basensequenz aufweist, oder
(2) eine Nukleinsäure, umfassend die folgenden (a) oder (b):
(a) eine Nukleinsäure, umfassend eine Basensequenz, die durch die in der SEQ ID Nr. 2 des Sequenzprotokolls gezeigten Basennummern 1-2090 dargestellt ist, oder
(b) eine Nukleinsäure, in der ein Teil der Basensequenz der genannten Basennummern 1-2090 deletiert, substituiert oder addiert ist, und die 80% Homologie mit genannter Basensequenz aufweist,
ist.

2. Verfahren zum Bestimmen, ob jemand vom Marfan-Syndrom betroffen ist, wobei die wie in Anspruch 1 definierte Sonde verwendet wird, **gekennzeichnet durch** das Bestimmen des Vorhandenseins/Fehlens einer Deletion oder Abscission in der Region p24.1 bis p14.2 auf dem Chromosom 3, oder **durch** Bestimmen des Vorhandenseins/Fehlens einer Deletion oder Punktmutation in einer Nukleinsäure, umfassend eine Basensequenz, die **durch** die in der SEQ ID Nr. 2 des Sequenzprotokolls gezeigten Basennummern 1-2090 dargestellt ist.

3. Verfahren nach Anspruch 2, das mit einem Verfahren, welches ein Nukleinsäurehybridisierungsverfahren verwendet, oder mit einem Verfahren, das eine Bestimmung der gesamten Basensequenz verwendet, erfolgt.

4. Verfahren nach Anspruch 3, wobei das Verfahren, das ein Nukleinsäurehybridisierungsverfahren verwendet, ein *in situ* Hybridisierungsverfahren oder ein Southern-Hybridisierungsverfahren ist.

5. Verfahren nach Anspruch 4, wobei das *in situ* Hybridisierungsverfahren ein *in situ* Fluoreszenz-Hybridisierungsverfahren ist.

## Revendications

1. Utilisation d'une sonde pour le diagnostic du syndrome de Marfan, la sonde étant
(1) un acide nucléique comprenant (a) ou (b) suivant :
(a) un acide nucléique comprenant une séquence de bases représentée par les numéros de base 1 à 180 000 montrés dans SEQ ID NO : 1 de la liste des séquences, ou
(b) un acide nucléique dans lequel une partie de la séquence de bases desdits numéros de base 1 à 180 000 est délétée, substituée ou ajoutée, et ayant 80 % d'homologie avec ladite séquence de bases, ou
(2) un acide nucléique comprenant (a) ou (b) suivant :
(a) un acide nucléique comprenant une séquence de bases représentée par les numéros de base 1 à 2 090 montrés dans SEQ ID NO : 2 de la liste des séquences, ou
(b) un acide nucléique dans lequel une partie de la séquence de bases desdits numéros de base 1 à 2 090 est délétée, substituée ou ajoutée, et ayant 80 % d'homologie avec ladite séquence de bases.

2. Procédé de détermination qu'une personne est ou non affectée par le syndrome de Marfan utilisant la sonde selon la revendication 1, **caractérisé par** la détermination de la présence/absence d'une délétion ou d'une abscission au niveau de la région de p24.1 à p14.2 sur le chromosome 3, ou par la détermination de la présence/absence d'une délétion ou d'une mutation ponctuelle dans un acide nucléique comprenant une séquence de bases représentée par les numéros de base 1 à 2 090 montrés dans SEQ ID NO : 2 de la liste des séquences.

3. Procédé selon la revendication 2, qui est conduit par un procédé utilisant un procédé d'hybridation d'acide nucléique ou par un procédé utilisant une détermination de la séquence de bases totale.

4. Procédé selon la revendication 3, dans lequel ledit procédé utilisant un procédé d'hybridation d'acide nucléique est un procédé d'hybridation *in situ* ou un procédé d'hybridation de Southern.

5. Procédé selon la revendication 4, dans lequel ledit procédé d'hybridation *in situ* est un procédé d'hybridation *in situ* par fluorescence.


### SEQUENCE LISTING

<110>President of Nagasaki University
<120>A PROBE FOR DIAGNOSIS OF MARFAN SYNDROME AND A SCREENING METHOD USING THE PROBE
<130> U 2003 P 450
<160>
<210> 1
   <211>180000
   <212>Nucleic Acid
   <213>Human Chromosome
<400> 1
<210> 2
   <211>2090
   <212>Amino Acid
   <213>Human Chromosome
<400> 2
   gttggcgagg agtttcctgt ttcccccgca gcgctgagtt gaagttgagt gagtcactcg 60
   cgcgcacgga gcgacgacac ccccgcgcgt gcacccgctc gggacaggag ccggactcct 120
   gtgcagcttc cctcggccgc cgggggcctc cccgcgcctc gccggcctcc aggcccctcc 180
   tggctggcga gcgggcgcca catctggccc gcacatctgc gctgccggcc cggcgcgggg 240
   tccggagagg gcgcggcgcg gagcgcagcc aggggtccgg gaaggcgccg tccgtgcgct 300
   gggggctcgg tctatgacga gcagcggggt ctgccatggg tcgggggctg ctcaggggcc 360
   tgtggccgct gcacatcgtc ctgtggacgc gtatcgccag cacgatccca ccgcacgttc 420
   agaagtcggt taataacgac atgatagtca ctgacaacaa cggtgcagtc aagtttccac 480
   aactgtgtaa attttgtgat gtgagatttt ccacctgtga caaccagaaa tcctgcatga 540
   gcaactgcag catcacctcc atctgtgaga agccacagga agtctgtgtg gctgtatgga 600
   gaaagaatga cgagaacata acactagaga cagtttgcca tgaccccaag ctcccctacc 660
   atgactttat tctggaagat gctgcttctc caaagtgcat tatgaaggaa aaaaaaaagc 720
   ctggtgagac tttcttcatg tgttcctgta gctctgatga gtgcaatgac aacatcatct 780
   tctcagaaga atataacacc agcaatcctg acttgttgct agtcatattt caagtgacag 840
   gcatcagcct cctgccacca ctgggagttg ccatatctgt catcatcatc ttctactgct 900
   accgcgttaa ccggcagcag aagctgagtt caacctggga aaccggcaag acgcggaagc 960
   tcatggagtt cagcgagcac tgtgccatca tcctggaaga tgaccgctct gacatcagct 1020
   ccacgtgtgc caacaacatc aaccacaaca cagagctgct gcccattgag ctggacaccc 1080
   tggtggggaa aggtcgcttt gctgaggtct ataaggccaa gctgaagcag aacacttcag 1140
   agcagtttga gacagtggca gtcaagatct ttccctatga ggagtatgcc tcttggaaga 1200
   cagagaagga catcttctca gacatcaatc tgaagcatga gaacatactc cagttcctga 1260
   cggctgagga gcggaagacg gagttgggga aacaatactg gctgatcacc gccttccacg 1320
   ccaagggcaa cctacaggag tacctgacgc ggcatgtcat cagctgggag gacctgcgca 1380
   agctgggcag ctccctcgcc cgggggattg ctcacctcca cagtgatcac actccatgtg 1440
   ggaggcccaa gatgcccatc gtgcacaggg acctcaagag ctccaatatc ctcgtgaaga 1500
   acgacctaac ctgctgcctg tgtgactttg ggctttccct gcgtctggac cctactctgt 1560
   ctgtggatga cctggctaac agtgggcagg tgggaactgc aagatacatg gctccagaag 1620
   tcctagaatc caggatgaat ttggagaatg ctgagtcctt caagcagacc gatgtctact 1680
   ccatggctct ggtgctctgg gaaatgacat ctcgctgtaa tgcagtggga gaagtaaaag 1740
   attatgagcc tccatttggt tccaaggtgc gggagcaccc ctgtgtcgaa agcatgaagg 1800
   acaacgtgtt gagagatcga gggcgaccag aaattcccag cttctggctc aaccaccagg 1860
   gcatccagat ggtgtgtgag acgttgactg agtgctggga ccacgaccca gaggcccgtc 1920
   tcacagccca gtgtgtggca gaacgcttca gtgagctgga gcatctggac aggctctcgg 1980
   ggaggagctg ctcggaggag aagattcctg aagacggctc cctaaacact accaaatagc 2040
   tcttatgggg caggctgggc atgtccaaag aggctgcccc tctcaccaaa 2090
<210> 3
   <211> 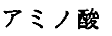
   <212> 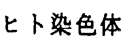
   <213>
<400> 3

### SEQUENCE LISTING

<110> Nagasaki university
   Institut national de la santé et de la recherche
<120> Probe for diagnosis of Marfan's syndrome and method of screening with the probe
<130> EPA-100508
<140> 05745933
   <141> 2005-05-27
<150> JP20040158099
   <151> 2004-05-27
<160> 3
<170> PatentIn version 3.3
<210> 1
   <211> 180000
   <212> DNA
   <213> human
<400> 1
<210> 2
   <211> 2090
   <212> DNA
   <213> human
<400> 2
   gttggcgagg agtttcctgt ttcccccgca gcgctgagtt gaagttgagt gagtcactcg 60
   cgcgcacgga gcgacgacac ccccgcgcgt gcacccgctc gggacaggag ccggactcct 120
   gtgcagcttc cctcggccgc cgggggcctc cccgcgcctc gccggcctcc aggcccctcc 180
   tggctggcga gcgggcgcca catctggccc gcacatctgc gctgccggcc cggcgcgggg 240
   tccggagagg gcgcggcgcg gagcgcagcc aggggtccgg gaaggcgccg tccgtgcgct 300
   gggggctcgg tctatgacga gcagcggggt ctgccatggg tcgggggctg ctcaggggcc 360
   tgtggccgct gcacatcgtc ctgtggacgc gtatcgccag cacgatccca ccgcacgttc 420
   agaagtcggt taataacgac atgatagtca ctgacaacaa cggtgcagtc aagtttccac 480
   aactgtgtaa attttgtgat gtgagatttt ccacctgtga caaccagaaa tcctgcatga 540
   gcaactgcag catcacctcc atctgtgaga agccacagga agtctgtgtg gctgtatgga 600
   gaaagaatga cgagaacata acactagaga cagtttgcca tgaccccaag ctcccctacc 660
   atgactttat tctggaagat gctgcttctc caaagtgcat tatgaaggaa aaaaaaaagc 720
   ctggtgagac tttcttcatg tgttcctgta gctctgatga gtgcaatgac aacatcatct 780
   tctcagaaga atataacacc agcaatcctg acttgttgct agtcatattt caagtgacag 840
   gcatcagcct cctgccacca ctgggagttg ccatatctgt catcatcatc ttctactgct 900
   accgcgttaa ccggcagcag aagctgagtt caacctggga aaccggcaag acgcggaagc 960
   tcatggagtt cagcgagcac tgtgccatca tcctggaaga tgaccgctct gacatcagct 1020
   ccacgtgtgc caacaacatc aaccacaaca cagagctgct gcccattgag ctggacaccc 1080
   tggtggggaa aggtcgcttt gctgaggtct ataaggccaa gctgaagcag aacacttcag 1140
   agcagtttga gacagtggca gtcaagatct ttccctatga ggagtatgcc tcttggaaga 1200
   cagagaagga catcttctca gacatcaatc tgaagcatga gaacatactc cagttcctga 1260
   cggctgagga gcggaagacg gagttgggga aacaatactg gctgatcacc gccttccacg 1320
   ccaagggcaa cctacaggag tacctgacgc ggcatgtcat cagctgggag gacctgcgca 1380
   agctgggcag ctccctcgcc cgggggattg ctcacctcca cagtgatcac actccatgtg 1440
   ggaggcccaa gatgcccatc gtgcacaggg acctcaagag ctccaatatc ctcgtgaaga 1500
   acgacctaac ctgctgcctg tgtgactttg ggctttccct gcgtctggac cctactctgt 1560
   ctgtggatga cctggctaac agtgggcagg tgggaactgc aagatacatg gctccagaag 1620
   tcctagaatc caggatgaat ttggagaatg ctgagtcctt caagcagacc gatgtctact 1680
   ccatggctct ggtgctctgg gaaatgacat ctcgctgtaa tgcagtggga gaagtaaaag 1740
   attatgagcc tccatttggt tccaaggtgc gggagcaccc ctgtgtcgaa agcatgaagg 1800
   acaacgtgtt gagagatcga gggcgaccag aaattcccag cttctggctc aaccaccagg 1860
   gcatccagat ggtgtgtgag acgttgactg agtgctggga ccacgaccca gaggcccgtc 1920
   tcacagccca gtgtgtggca gaacgcttca gtgagctgga gcatctggac aggctctcgg 1980
   ggaggagctg ctcggaggag aagattcctg aagacggctc cctaaacact accaaatagc 2040
   tcttatgggg caggctgggc atgtccaaag aggctgcccc tctcaccaaa 2090
<210> 3
   <211> 567
   <212> PRT
   <213> human
<400> 3
   Met Gly Arg Gly Leu Leu Arg Gly Leu Trp Pro Leu His Ile Val Leu
   1               5                   10                  15
   Trp Thr Arg Ile Ala Ser Thr Ile Pro Pro His Val Gln Lys Ser Val
               20                  25                  30
   Asn Asn Asp Met Ile Val Thr Asp Asn Asn Gly Ala Val Lys Phe Pro
   35 40 45
   Gln Leu Cys Lys Phe Cys Asp Val Arg Phe Ser Thr Cys Asp Asn Gln
   50 55 60
   Lys Ser Cys Met Ser Asn Cys Ser Ile Thr Ser Ile Cys Glu Lys Pro
   65 70 75 80
   Gln Glu Val Cys Val Ala Val Trp Arg Lys Asn Asp Glu Asn Ile Thr
   85 90 95
   Leu Glu Thr Val Cys His Asp Pro Lys Leu Pro Tyr His Asp Phe Ile
   100 105 110
   Leu Glu Asp Ala Ala Ser Pro Lys Cys Ile Met Lys Glu Lys Lys Lys
   115 120 125
   Pro Gly Glu Thr Phe Phe Met Cys Ser Cys Ser Ser Asp Glu Cys Asn
   130 135 140
   Asp Asn Ile Ile Phe Ser Glu Glu Tyr Asn Thr Ser Asn Pro Asp Leu
   145 150 155 160
   Leu Leu Val Ile Phe Gln Val Thr Gly Ile Ser Leu Leu Pro Pro Leu
   165 170 175
   Gly Val Ala Ile Ser Val Ile Ile Ile Phe Tyr Cys Tyr Arg Val Asn
   180 185 190
   Arg Gln Gln Lys Leu Ser Ser Thr Trp Glu Thr Gly Lys Thr Arg Lys
   195 200 205
   Leu Met Glu Phe Ser Glu His Cys Ala Ile Ile Leu Glu Asp Asp Arg
   210 215 220
   Ser Asp Ile Ser Ser Thr Cys Ala Asn Asn Ile Asn His Asn Thr Glu
   225 230 235 240
   Leu Leu Pro Ile Glu Leu Asp Thr Leu Val Gly Lys Gly Arg Phe Ala
   245 250 255
   Glu Val Tyr Lys Ala Lys Leu Lys Gln Asn Thr Ser Glu Gln Phe Glu
   260 265 270
   Thr Val Ala Val Lys Ile Phe Pro Tyr Glu Glu Tyr Ala Ser Trp Lys
   275 280 285
   Thr Glu Lys Asp Ile Phe Ser Asp Ile Asn Leu Lys His Glu Asn Ile
   290 295 300
   Leu Gln Phe Leu Thr Ala Glu Glu Arg Lys Thr Glu Leu Gly Lys Gln
   305                 310                 315                 320
   Tyr Trp Leu Ile Thr Ala Phe His Ala Lys Gly Asn Leu Gln Glu Tyr
                   325                 330                 335
   Leu Thr Arg His Val Ile Ser Trp Glu Asp Leu Arg Lys Leu Gly Ser
               340                 345                 350
   Ser Leu Ala Arg Gly Ile Ala His Leu His Ser Asp His Thr Pro Cys
           355                 360                 365
   Gly Arg Pro Lys Met Pro Ile Val His Arg Asp Leu Lys Ser Ser Asn
       370                 375                 380
   Ile Leu Val Lys Asn Asp Leu Thr Cys Cys Leu Cys Asp Phe Gly Leu
   385                 390                 395                 400
   Ser Leu Arg Leu Asp Pro Thr Leu Ser Val Asp Asp Leu Ala Asn Ser
                   405                 410                 415
   Gly Gln Val Gly Thr Ala Arg Tyr Met Ala Pro Glu Val Leu Glu Ser
               420                 425                 430
   Arg Met Asn Leu Glu Asn Ala Glu Ser Phe Lys Gln Thr Asp Val Tyr
           435                 440                 445
   Ser Met Ala Leu Val Leu Trp Glu Met Thr Ser Arg Cys Asn Ala Val
       450                 455                 460
   Gly Glu Val Lys Asp Tyr Glu Pro Pro Phe Gly Ser Lys Val Arg Glu
   465                 470                 475                 480
   His Pro Cys Val Glu Ser Met Lys Asp Asn Val Leu Arg Asp Arg Gly
                   485                 490                 495
   Arg Pro Glu Ile Pro Ser Phe Trp Leu Asn His Gln Gly Ile Gln Met
               500                 505                 510
   Val Cys Glu Thr Leu Thr Glu Cys Trp Asp His Asp Pro Glu Ala Arg
           515                 520                 525
   Leu Thr Ala Gln Cys Val Ala Glu Arg Phe Ser Glu Leu Glu His Leu
       530                 535                 540
   Asp Arg Leu Ser Gly Arg Ser Cys Ser Glu Glu Lys Ile Pro Glu Asp
   545                 550                 555                 560
   Gly Ser Leu Asn Thr Thr Lys
                   565